# FASCICULE DE BREVET EUROPEEN

(11) **EP 2 537 555 B1**
(45) Date de publication et mention de la délivrance du brevet: **01.05.2013**
(21) Numéro de dépôt: 12164451.2
(22) Date de dépôt: 17.04.2012
(51) Int. Cl.: A61N 1/375, A61N 1/05

(54) **Implant intracardiaque autonome de type leadless à élément de fixation désolidarisable**
Autonomes Herzimplantat vom Typ leadless mit einem nicht ablösbaren Befestigungselement
Leadless autonomous intracardiac implant with disengageable attachment element

(30) Priorité: 24.06.2011 FR 1155622
(43) Date de publication de la demande: 26.12.2012
(73) Titulaire: Sorin CRM SAS, 92140 Clamart Cedex (FR)
(72) Inventeur: Régnier, Willy, 91160 Longjumeau (FR); Deterre, Martin, 75013 Paris (FR); Poussin, Patrice, 92500 Rueil Malmaison (FR)
(74) Mandataire: Dupuis-Latour, Dominique

(56) Documents cités:
- US-A- 5 551 427
- US-A- 6 141 588
- US-A1- 2002 165 589
- US-B1- 6 354 299

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance trans-pulmonaire ou d'impédance intracardiaque).

L'invention concerne plus particulièrement ceux de ces dispositifs qui mettent en oeuvre des implants autonomes dépourvus de toute liaison physique à un dispositif principal implanté (tel qu'un boîtier de générateur d'impulsions de stimulation) ou non implanté (périphérique externe tel que programmateur ou dispositif de *monitoring* pour le suivi à distance du patient). La communication est alors du type communication sans fil.

Ces implants autonomes sont dénommées pour cette raison "implants *leadless*", pour les distinguer des électrodes ou des capteurs disposés à l'extrémité distale d'une sonde (*lead*)*,* cette sonde étant parcourue sur toute sa longueur par un ou plusieurs conducteurs reliant par voie galvanique l'électrode ou le capteur à un générateur connecté à l'extrémité opposée, proximale, de la sonde.

De tels implants *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces implants *leadless* peuvent être notamment des implants épicardiques, fixés à la paroi extérieure du coeur, ou bien des implants endocavitaires, fixés à la paroi intérieure d'une cavité ventriculaire ou auriculaire. Leur fixation à la paroi cardiaque se fait habituellement au moyen d'une vis d'ancrage hélicoïdale saillante, prolongeant axialement le corps de l'implant et destinée à pénétrer dans le tissu cardiaque par vissage au site d'implantation.

Un tel implant comprend des circuits de détection/stimulation pour recueillir des potentiels de dépolarisation du myocarde et/ou pour appliquer des impulsions de stimulation au site d'implantation, au moyen d'électrodes appropriées portées par cet implant. Il peut également incorporer un ou plusieurs capteurs permettant de mesurer localement la valeur d'un paramètre tel que le niveau d'oxygène dans le sang, la pression cardiaque endocavitaire, l'accélération de la paroi cardiaque, l'accélération du patient comme indicateur de l'activité etc. Bien entendu, pour permettre l'échange de données à distance, les implants *leadless* incorporent également des moyens émetteurs/récepteurs de communication sans fil.

L'invention n'est toutefois pas limitée à un type particulier d'implant, et elle est applicable indifféremment à tout type d'implant *leadless,* quel que soit sa destination fonctionnelle.

À cet égard, le US2002/0165589 A1 décrit un implant comprenant des moyens d'ancrage à la paroi gastrique, montés sur une embase pouvant être couplée à un boîtier détachable et remplaçable comprenant des circuits électroniques.

La source d'énergie est l'un des points faibles majeurs des implants lea *dless,* car, étant autonomes, il n'est pas possible de leur transmettre de l'énergie par un conducteur de sonde. Même si des techniques de récupération d'énergie ont été proposées, à ce jour seuls les systèmes d'alimentation par piles sont véritablement opérationnels. Mais compte tenu des contraintes de volume très restrictives, l'autonomie de ces piles est limitée, de sorte que les implants *leadless* actuellement proposés présentent une durée de vie limitée, de l'ordre de six mois à deux ans, et doivent donc être remplacés régulièrement.

Le remplacement d'un implant *leadless,* outre la réitération fréquente d'une opération particulièrement invasive, entraîne plusieurs difficultés :
- en premier lieu, l'ancien site d'implantation, qui était peut-être optimal (notamment s'il a été déterminé à la suite d'un *mapping*) n'est pas retrouvable facilement ;
- d'autre part, des traumatismes supplémentaires sont causés aux tissus par le retrait de l'ancien implant et la fixation du nouveau ;
- enfin, lorsque l'implant en fin de vie ne peut pas être retiré et doit être laissé en place, il constitue un corps étranger invasif et parasite, ce qui peut s'avérer très problématique au fil des ans et des implantations successives.

Les problèmes ci-dessus se posent d'ailleurs quelle que soit la cause du remplacement de l'implant *leadless* : défaut d'un circuit électronique, remplacement par une version plus récente, élément générant une infection, etc.

De surcroît, l'introduction jusqu'au site d'implantation d'un implant *leadless* de dimension relativement importante nécessite des outils de taille adaptée, dont l'utilisation peut s'avérer traumatique pour le patient.

Enfin, dans tous les systèmes jusqu'à présent proposés, l'axe de fixation de l'implant *leadless* (typiquement, l'axe de la vis d'ancrage) est le même que l'axe d'introduction de l'implant. Pour un implant endocavitaire, cela signifie que cet élément de fixation se situe au bout du corps cylindrique allongé constituant le corps de l'implant, qui doit donc être obligatoirement fixé perpendiculairement à la paroi cardiaque. Cette configuration augmente l'invasivité du système implanté par rapport au fonctionnement du coeur, notamment du fait de l'interférence plus importante avec le flux sanguin et le mouvement des parois cardiaques.

Le but de l'invention est de proposer un dispositif de type implant *leadless* permettant de pallier ces différents inconvénients en permettant notamment, lorsque l'implant doit être renouvelé :
- de réutiliser le site d'implantation initial ;
- de minimiser les traumatismes sur les tissus au site d'implantation ;
- si l'implant doit être retiré, de permettre ce retrait sans laisser subsister sur le site d'implantation d'éléments volumineux ;
- de minimiser l'invasivité lors de l'implantation ; et
- d'augmenter la liberté de conception de la forme de l'implant, notamment en évitant de concevoir un élément allongé implantable dont après implantation la plus grande dimension serait perpendiculaire à la paroi cardiaque.

Comme dans le US2002/0165589 A1 précité, l'implant est dissocié en deux éléments distincts, avec :
- un premier élément ou "embase", dédié à la fixation à la paroi cardiaque, comprenant les moyens d'ancrage usuels tels que vis, harpon, crochet ou autre ; et
- un second élément ou "capsule" incorporant les principaux organes actifs de l'implant (électronique et sources d'énergie),
- les deux éléments étant couplés de manière réversible par un système de fixation tel que clip, vis, baïonnette ou autre.

Lors de l'implantation du système, le premier élément (embase) sera fixé en premier sur le site choisi. Ensuite, le second élément (capsule) sera inséré puis fixé à l'embase grâce au système de couplage.

De façon caractéristique, le système de couplage comprend des pattes saillantes disposées à deux extrémités opposées de l'embase de manière à définir des surfaces de contact en vis-à-vis servant à épouser la forme de surfaces de contact conjuguées de la capsule.

Comme on le verra par la suite, un tel implant selon l'invention peut être adapté aussi bien à des implants *leadless* endocavitaires, de forme allongée (dont la longueur est supérieure au diamètre), qu'à des implants épicardiques de forme plus plate (dont le diamètre est supérieur à la longueur).

Quand une intervention est nécessaire pour l'échange de l'implant (pile épuisée, système obsolète, etc.), il suffit de détacher la capsule de l'embase, et d'en installer une nouvelle à sa place. Ainsi, le site de stimulation est préservé et les tissus cardiaques ne subissent pas de traumatisme supplémentaire dû à l'extraction et à la réimplantation des moyens d'ancrage.

De plus, un tel concept accroît notablement la modularité et l'adaptabilité des implants *leadless.*

Ainsi, il est par exemple possible de décliner une famille de capsules pouvant s'adapter sur une embase standard équipée des moyens d'ancrage, sans remettre en cause l'implantation du système. Inversement, il est possible d'envisager une famille d'embases spécifiquement conçues pour l'implantation en différents endroits du coeur, et susceptibles de recevoir un même type de capsule ou une même famille de capsules.

Plus précisément, le dispositif de l'invention comprend, qui est du type divulgué par le US2002/0165589 A1 précité, c'st-à-dire comprenant deux éléments distincts solidarisables entre eux et séparables de manière réversible, avec une capsule étanche logeant des circuits électroniques, et une embase comportant une platine présentant une face extérieure portant des moyens d'ancrage à une paroi d'un organe d'un patient, ainsi qu'une face intérieure formant support pour la capsule et portant des moyens de couplage mécanique à la capsule, est caractérisé par le fait que les moyens de couplage mécanique de l'embase à la capsule comprennent des pattes saillantes disposées à deux extrémités opposées de la platine sur la face intérieure de celle-ci, ces pattes étant sensiblement parallèles et définissant sur leurs faces en vis-à-vis des surfaces de contact aptes à épouser la forme de surfaces de contact conjuguées de la capsule.

Les pattes peuvent être élastiquement déformables, les surfaces de contact de l'embase comportant alors des concavités en vis-à-vis aptes à épouser la forme de surfaces de contact conjuguées convexes de la capsule, de manière à permettre un clipsage réversible de la capsule sur l'embase.

Les pattes peuvent être rigides, les surfaces de contact de l'embase comportant par exemple un taraudage apte à venir en prise avec des surfaces de contact conjuguées filetées de la capsule, de manière à permettre un vissage réversible de la capsule sur l'embase. En variante, les pattes comportent une encoche courbe apte à venir en prise avec des doigts de couplage homologues de la capsule, de manière à permettre un emboîtement réversible de type fixation à baïonnette de la capsule sur l'embase. Dans ce dernier cas, l'embase comporte en outre des moyens de sollicitation élastique de la capsule dans le sens axial de manière à permettre un verrouillage en position de la capsule fixée sur l'embase.

Dans un premier mode de mise en oeuvre, la capsule comporte un corps en forme de surface de révolution dont l'axe est orienté parallèlement à l'axe des moyens d'ancrage, et ce corps comporte, sur sa face tournée du côté de l'embase, au moins un support saillant portant une surface d'électrode apte à venir en contact avec les tissus du patient lorsque la capsule est montée sur l'embase.

Il peut alors être prévu une paroi latérale du support saillant, apte à venir en contact avec un rebord de la platine de manière à assurer un blocage en rotation axiale de la capsule par rapport à l'embase.

Dans une variante de réalisation, la capsule comporte un corps pourvu d'électrodes couplées à des contacts disposés sur la partie intérieure de l'embase, ces contacts étant eux-mêmes reliés à des électrodes formées sur la partie extérieure de l'embase et aptes à venir en contact avec la paroi de l'organe du patient.

Dans une forme particulière de mise en oeuvre, le corps comporte, sur sa face opposée à celle tournée du côté de l'embase, un couvercle portant une surface conductrice formant électrode de masse, apte à venir en contact avec un fluide corporel de l'organe du patient lorsque la capsule est montée sur l'embase.

Ce premier mode de mise en oeuvre se prête en particulier à une réalisation où le corps comporte, sur sa face opposée à celle tournée du côté de l'embase, des moyens de fixation à un élément additionnel séparable du corps sans désolidarisation du corps et de l'embase, la capsule étant formée par l'empilement du corps et de l'élément additionnel. L'élément additionnel peut notamment loger une batterie d'alimentation des circuits électroniques logés dans le corps.

Dans un deuxième mode de mise en oeuvre, la capsule comporte un corps en forme de surface de révolution dont l'axe est orienté perpendiculairement à l'axe des moyens d'ancrage, et dans lequel le corps comporte, sur au moins une région d'extrémité axiale, une bague annulaire portant une surface d'électrode apte à venir en contact avec les tissus du patient lorsque la capsule est montée sur l'embase.

Dans tous les cas, la capsule et/ou l'embase peuvent être avantageusement pourvus de marqueurs radio-opaques, afin de faciliter ultérieurement les opérations d'extraction de la capsule puis de mise en place d'une nouvelle capsule sur l'embase, restée implantée *in situ.* ◊

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 illustre de façon schématique un ensemble de dispositifs médicaux comprenant notamment des dispositifs *leadless* implantées au sein du corps d'un patient.
La Figure 2 montre plus précisément la manière d'implanter ces dispositifs *leadless* sur la paroi interne ou externe du myocarde.
La Figure 3 est un schéma par blocs fonctionnels montrant les différents étages constitutifs d'un implant *leadless.*
Les Figures 4a à 4e illustrent un premier mode de réalisation de l'implant *leadless* selon l'invention, pour une capsule épicardique avec fixation par clipsage de la capsule à l'embase.
Les Figures 5a et 5b illustrent un deuxième mode de réalisation de l'implant *leadless* selon l'invention, pour une capsule endocavitaire avec fixation par clipsage de la capsule à l'embase.
Les Figures 6a et 6b illustrent un troisième mode de réalisation de l'implant *leadless* selon l'invention, pour une capsule épicardique avec fixation par vissage de la capsule sur l'embase.
Les Figures 7a à 7c illustrent un quatrième mode de réalisation de l'implant *leadless* selon l'invention, pour une capsule épicardique avec fixation par un système à baïonnette de la capsule à l'embase.

On va maintenant décrire divers exemples de réalisation de l'invention.

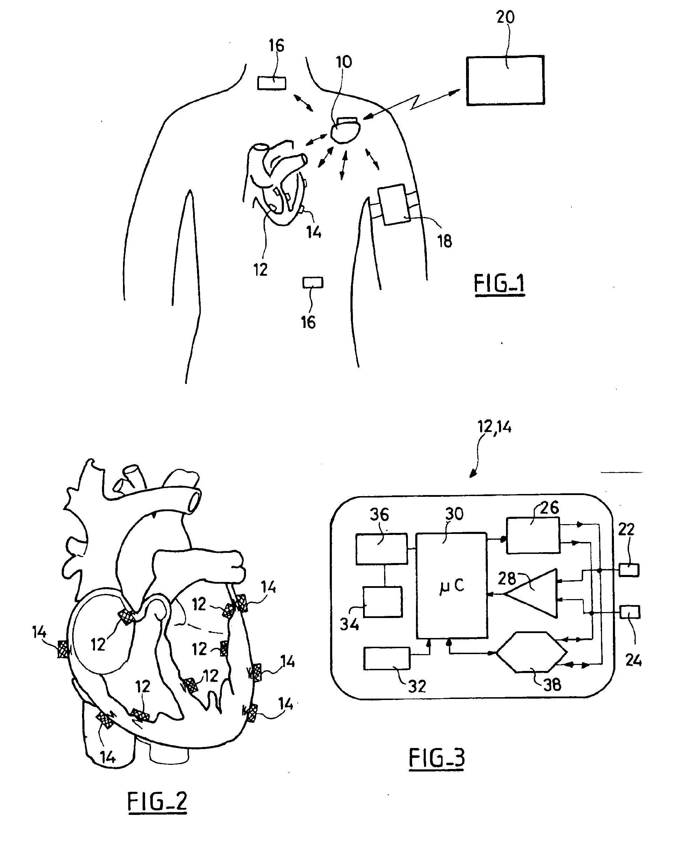

Sur la Figure 1 on a illustré un ensemble de dispositifs médicaux implantés au sein du corps d'un patient. Le patient est équipé par exemple d'un dispositif 10 tel qu'un défibrillateur/stimulateur/resynchroniseur implanté, ou un défibrillateur de type sous-cutané, ou encore un enregistreur longue durée. Ce dispositif 10 est le dispositif maître d'un réseau comportant une pluralité de dispositifs esclaves 12 à 18 avec lesquels il est susceptible d'entrer en communication par voie HBC (*Human Body Communication*, communication par voie intracorporelle). Ceux-ci peuvent notamment inclure des dispositifs intracardiaques 12 ou épicardiques 14 implantés directement sur le coeur du patient, d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif maître 10 peut également être utilisé en tant que passerelle avec le monde extérieur pour communiquer avec un périphérique externe 20 du type programmateur ou dispositif de télétransmission de données avec lequel il pourra communiquer notamment par télémétrie RF.

Chacun des dispositifs 10 à 18 est muni d'au moins un couple d'électrodes qui se trouvent en contact direct avec les tissus du corps pour les dispositifs implantés, ou en contact avec la peau pour le dispositif externe 18.

Sur la Figure 2, on a représenté des exemples de dispositifs de type *leadless* implantés soit sur la partie intérieure du myocarde, dans une cavité auriculaire ou ventriculaire (implants endocavitaires 12), soit sur une paroi externe de ce même myocarde (implants épicardiques 14). Ces dispositifs sont fixés à la paroi cardiaque au moyen d'une vis d'ancrage saillante destinée à pénétrer dans le tissu cardiaque par vissage au site d'implantation. La vis peut être soit une vis passive, ne servant qu'à la fixation de l'implant, soit une vis active, servant à recueillir les signaux de dépolarisation se propageant dans les tissus du myocarde et/ou à délivrer des impulsions de stimulation au site d'implantation, de façon localisée.

La Figure 3 illustre de façon schématique les différents circuits internes des implants 12 ou 14.

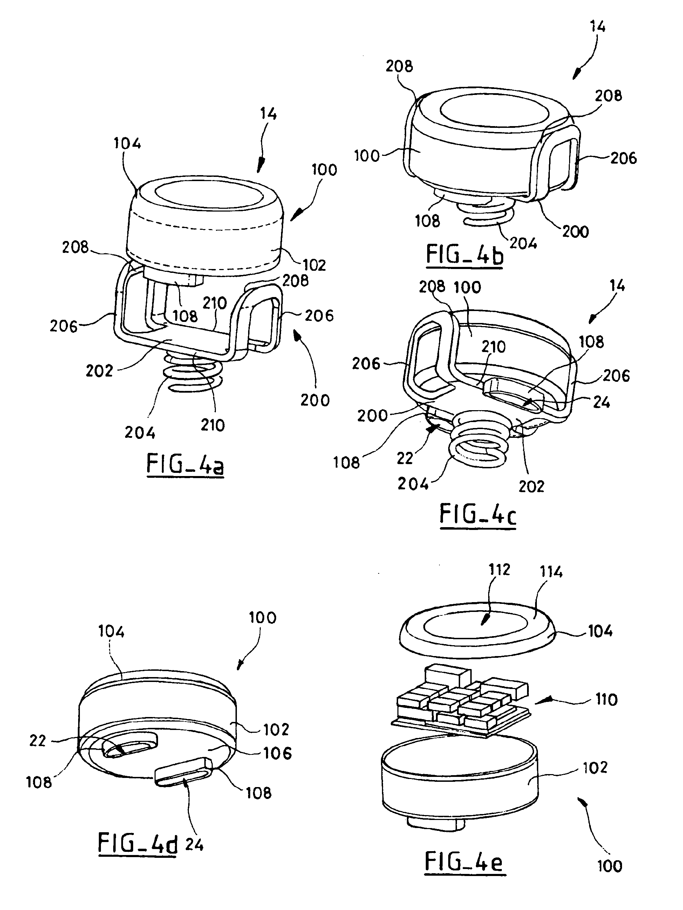

Chaque implant comporte un couple d'électrodes 22, 24 reliées à un circuit 26 générateur d'impulsions de stimulation (pour un implant actif incorporant cette fonction) et/ou à un circuit de détection 28 servant au recueil des potentiels de dépolarisation recueillis entre les électrodes 22 et 24. Un circuit central 30 inclut l'ensemble de l'électronique permettant de piloter les diverses fonctions de l'implant, la mémorisation des signaux recueillis, etc. Il comprend un microcontrôleur et un oscillateur générant les signaux d'horloge nécessaires au fonctionnement du microcontrôleur et à la communication. Il peut également contenir un convertisseur analogique/numérique et une mémoire de stockage numérique. L'implant peut également être pourvu d'un capteur 32 tel qu'un capteur d'accélération, de pression, un capteur hémodynamique, de température, de saturation en oxygène, etc. L'implant comprend une petite batterie ou un circuit de récupération d'énergie 34 alimentant l'ensemble des circuits via un étage de gestion d'énergie 36. Les électrodes 22 et 24 sont également reliées à un circuit modulateur/ démodulateur 38 couplé au circuit processeur central 30 et apte à émettre et/ou recevoir des impulsions servant à la communication sans fil HBC. Ainsi, selon que les circuits de stimulation (module 26) et de recueil (module 28) sont présents ou non, les électrodes 22, 24 peuvent assurer une simple, double ou triple fonction, à savoir : stimulation et/ou recueil des potentiels cardiaques (le cas échéant) ; et/ou transmission des informations suivies par le capteur 32 (le cas échéant) ; et émission/réception pour la communication HBC (en tout état de cause). De façon caractéristique de l'invention, et comme illustré sur les Figures 4 à 7, l'implant 12 ou 14 comprend une capsule 100 montée sur une embase 200 au moyen d'un couplage réversible.

Les Figures 4a à 4e illustrent un premier mode de réalisation de l'implant selon l'invention, pour une capsule épicardique avec fixation par clipsage de la capsule à l'embase.

Dans ce mode de réalisation, la capsule 100 comprend un corps cylindrique aplati, typiquement de quelques millimètres d'épaisseur et de 8 à 12 mm de diamètre, fermé par un couvercle 104 à sa partie supérieure (c'est-à-dire du côté opposé à la paroi sur laquelle l'implant est destiné à être fixé).

Le corps de la capsule 100 peut être réalisé en titane, selon une technologie conventionnelle d'emboutissage d'une fine lame de titane implantable conforme à ISO 5832-2, ou en tout autre métal biocompatible.

En variante, la capsule peut être réalisée en une matière plastique biocompatible, par moulage ou toute autre technique permettant d'encapsuler les composants internes logés dans le corps de la capsule. Cette matière plastique peut par exemple être un *Tecothane* (marque déposée), qui est un polyuréthanne thermoplastique à base de polyéther aromatique de grade médical, radio-opaque ou non.

Sur sa face inférieure 106 (voir notamment Figure 4d) la capsule 100 comprend deux éléments saillants 108 dont la surface destinée à venir en contact avec les tissus cardiaques, porte des électrodes 22, 24 de détection/stimulation/défibrillation. Ces électrodes présentent une surface de quelques millimètres carrés à quelques dizaines de millimètres carrés. Comme illustré Figure 4e, la capsule 100 enferme un circuit 110 portant les éléments actifs, la pile d'alimentation, les capteurs, les liaisons aux électrodes, etc. Ce circuit 110 est logé dans le corps 102 de la capsule qui est fermé de manière étanche par le couvercle 104, par exemple par soudure au corps cylindrique 102. La partie centrale du couvercle 104 peut éventuellement porter une surface conductrice 112 formant électrode de masse, isolée du reste du corps par une bague périphérique 114 en matériau isolant.

L'embase 200 comprend une platine 202 sur laquelle vient en appui la surface inférieure 106 du corps 102. La face inférieure de la platine 202 porte le moyen d'ancrage à la paroi cardiaque, en l'espèce une vis hélicoïdale 204 de 2 à 3 mm de diamètre.

Du côté opposé, c'est-à-dire du côté tourné vers la capsule 100, la platine 202 est pourvue à chacune de ses extrémités d'une patte 206 élastiquement déformable. L'ensemble platine 202/pattes 206 est avantageusement réalisé en *Tecothane* (marque déposée) de grade médical. Les pattes 206 s'étendent vers le haut de façon sensiblement parallèle et présentent dans leurs faces en vis-à-vis une légère concavité 208, épousant la forme de la surface périphérique conjuguée, légèrement convexe, du corps 102 de la capsule, de manière à assurer la rétention de la capsule 100 par clipsage du corps 102 entre les deux pattes 206 élastiquement déformables.

Avantageusement, comme illustré sur la Figure 4c qui montre l'ensemble capsule 100/embase 200 en configuration de stimulation, les éléments saillants 108 de la capsule 100, qui portent les électrodes respectives 22 et 24, viennent s'ajuster sans jeu ou avec un très faible jeu contre les rebords 210 de la partie centrale de la platine 202, ce qui permet de bloquer en rotation la capsule 100 par rapport à l'embase 200 et évite ainsi toute modification des zones de stimulation, c'est-à-dire des zones de la paroi cardiaque situées en contact avec chacune des deux électrodes 22 et 24. On notera que, pour que le contact avec la paroi cardiaque soit effectif, l'épaisseur des éléments saillants 108 doit être supérieure à l'épaisseur de la partie centrale de la platine 202.

Dans une variante de réalisation, les électrodes de stimulation (et/ou de détection) en contact avec les tissus à stimuler sont formées sur l'embase et non sur la capsule. Dans ce cas, le corps 102 de la capsule est pourvu d'électrodes couplées à des contacts disposés sur la partie intérieure de l'embase 200, contacts eux-mêmes reliés à des électrodes sur la partie extérieure de l'embase, en contact avec les tissus. Ce mode de réalisation rend en particulier possible une stimulation via les moyens de fixations de l'embase sur les tissus, par exemple par la vis d'ancrage 204.

Selon un autre aspect de l'invention, l'embase 200 et/ou la capsule 100 peuvent être pourvus de marqueurs radio-opaques, afin de faciliter ultérieurement les opérations d'extraction de la capsule puis de mise en place d'une nouvelle capsule sur l'embase, restée implantée *in situ.*

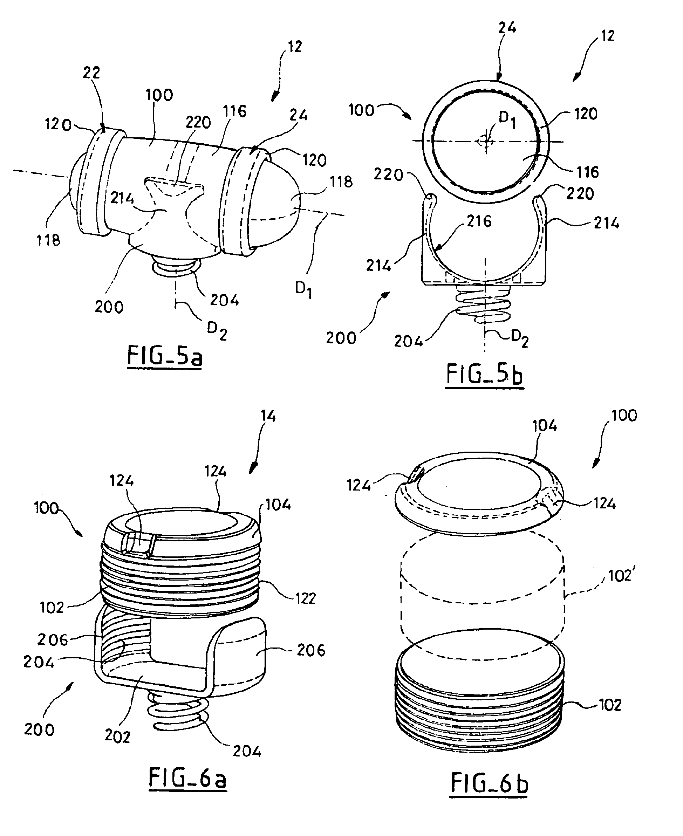

Les Figures 5a et 5b illustrent un deuxième mode de réalisation de l'implant selon l'invention, pour une capsule endocavitaire avec fixation par clipsage de la capsule à l'embase.

Dans ce mode de réalisation, la capsule 100 se présente sous forme d'un élément oblong avec un corps allongé terminé par deux extrémités en forme d'ogive 118. La capsule présente une dimension longitudinale de l'ordre de 10 mm, pour un diamètre maximal de quelques millimètres.

On notera que, dans cette configuration, le corps de la capsule 100 est disposé avec son axe D₁ orienté dans un sens sensiblement parallèle à la paroi cardiaque, c'est-à-dire perpendiculaire à l'axe D₂ des moyens d'ancrage 204 de l'embase 200 - à la différence du mode de réalisation précédent illustré Figures 4a à 4e, où ces deux axes étaient alignés et confondus.

L'embase comporte deux pattes élastiques 214 définissant, côté intérieur, une surface concave 216 homologue de la surface extérieure 116 de la capsule 100. La longueur des pattes 214 est telle que les extrémités 220 de celles-ci se situent au-delà de la région diamétrale de la capsule 100, de manière à pouvoir assurer la rétention en place de celle-ci après que cette dernière ait été emboîtée dans l'embase.

La capsule 100 est munie de part et d'autre de l'embase 200 de deux bagues annulaires 120, disposées au voisinage des extrémités 118 en forme d'ogive et portant les électrodes 22, 24 en forme de surfaces conductrices sur toute la périphérie des bagues 120.

Cette configuration permet un contact avec les tissus cardiaques quelle que soit la manière dont la capsule 100, qui présente une symétrie de révolution autour de l'axe D₁, a été emboîtée sur l'embase 200.Elle permet en outre de prévoir un écartement relativement important entre les deux électrodes 22 et 24, favorable à une stimulation efficace.

On notera également que le faible diamètre (quelques millimètres) de la capsule permet une introduction peu traumatique jusqu'au site d'implantation. La capsule est ensuite tournée d'un quart de tour au moment de son emboîtement sur l'embase 200, de manière à orienter perpendiculairement l'axe D₁ de la capsule par rapport à l'axe D₂ de l'embase et de la vis d'ancrage.

Dans une variante (non illustrée), le système comporte plusieurs, typiquement deux, embases fixées côte-à-côte sur la paroi cardiaque et sur lesquelles vient s'emboîter une capsule unique. L'implant est ainsi fixé à la paroi en plusieurs, typiquement deux, endroits, ce qui permet d'augmenter la surface en contact et procure une plus grande liberté en ce qui concerne le choix des sites de stimulation, ainsi qu'une meilleure tenue mécanique du fait de l'absence d'axe de rotation ou de flexion privilégié.

Les Figures 6a et 6b illustrent un troisième mode de réalisation de l'implant selon l'invention, pour une capsule épicardique avec fixation par vissage de la capsule sur l'embase.

Dans cette configuration, les pattes 206 de l'embase 200 sont pourvues sur leur face intérieure d'un taraudage 224 apte à coopérer avec un filetage homologue 122 réalisé sur la surface extérieure du corps 102 de la capsule 100. La mise en place de la capsule sur l'embase se fait alors par vissage, le couvercle 104 de la capsule étant avantageusement pourvu d'évidements 124 permettant d'engager un outil d'entraînement en rotation de la capsule.

Sur la Figure 6b on a illustré une variante dans laquelle l'implant peut comporter une pluralité de corps empilables tels que 102, 102' : le corps 102 comprend les moyens de fixation à l'embase et enferme par exemple l'ensemble des circuits électroniques, tandis que le corps 102' superposé au corps 102 contient la pile d'alimentation, de manière à pouvoir être remplacé en laissant en place le corps 102 monté sur l'embase.

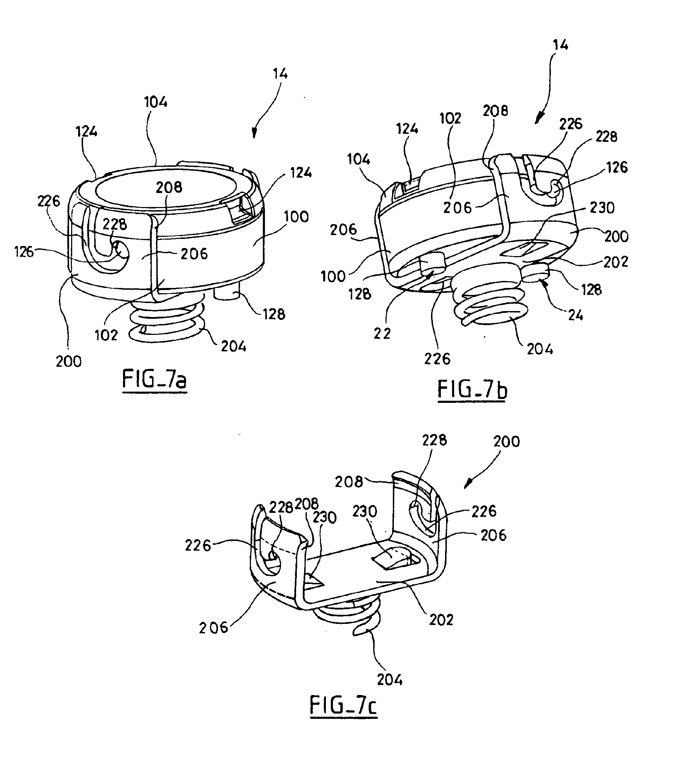

Les Figures 7a à 7c illustrent un quatrième mode de réalisation de l'implant selon l'invention, pour une capsule épicardique avec fixation par un système à baïonnette de la capsule à l'embase.

Dans cette configuration, les pattes 206 portent une encoche de guidage courbe 226, 228 coopérant avec un doigt de couplage 126 réalisé sur la paroi latérale de la capsule 100. La mise en place se fait à la manière d'une baïonnette d'un culot de lampe électrique, par un mouvement poussé-tourné. Le maintien en place est assuré par des éléments élastiques 230 formés sur la platine 202. Ces éléments élastiques assurent, une fois le mouvement de mise en place achevé, le plaquage du doigt de couplage 126 dans le fond 228 de l'encoche de guidage courbe 226.

Dans ce mode de réalisation, les électrodes de stimulation 22, 24 sont portées par des éléments saillants tels que 128 formés sur la surface inférieure du corps de la capsule 100 et destinés à venir en contact avec les tissus cardiaques une fois la capsule emboîtée sur l'embase.

## Revendications

1. Un dispositif médical actif intracorporel autonome (12, 14), comportant des moyens (204) d'ancrage à une paroi d'un organe d'un patient comprenant deux éléments distincts solidarisables entre eux et séparables de manière réversible, avec :
- une capsule étanche (100) logeant des circuits électroniques (110), et
- une embase (200) comportant une platine (202) présentant une face extérieure portant lesdits moyens d'ancrage (204), et une face intérieure formant support pour la capsule et portant des moyens de couplage mécanique à la capsule,
**caractérisé en ce que** les moyens de couplage mécanique de l'embase à la capsule comprennent des pattes saillantes (206) disposées à deux extrémités opposées de la platine (202) sur la face intérieure de celle-ci, ces pattes (206) étant sensiblement parallèles et définissant sur leurs faces en vis-à-vis des surfaces de contact aptes à épouser la forme de surfaces de contact conjuguées de la capsule.

2. Le dispositif de la revendication 1, dans lequel les pattes (206) sont élastiquement déformables, et dans lequel les surfaces de contact de l'embase comportent des concavités en vis-à-vis (208) aptes à épouser la forme de surfaces de contact conjuguées convexes (102) de la capsule, de manière à permettre un clipsage réversible de la capsule sur l'embase.

3. Le dispositif de la revendication 1, dans lequel les pattes (206) sont des pattes rigides, et dans lequel les surfaces de contact de l'embase comportent un taraudage (224) apte à venir en prise avec des surfaces de contact conjuguées filetées (122) de la capsule, de manière à permettre un vissage réversible de la capsule sur l'embase.

4. Le dispositif de la revendication 1, dans lequel les pattes sont des pattes rigides, et dans lequel les pattes comportent une encoche courbe (226, 228) apte à venir en prise avec des doigts de couplage homologues (126) de la capsule, de manière à permettre un emboîtement réversible de type fixation à baïonnette de la capsule sur l'embase, l'embase comportant en outre des moyens (230) de sollicitation élastique de la capsule dans le sens axial de manière à permettre un verrouillage en position de la capsule fixée sur l'embase.

5. Le dispositif de la revendication 1, dans lequel la capsule (100) comporte un corps (102) pourvu d'électrodes couplées à des contacts disposés sur la partie intérieure de l'embase (200), ces contacts étant eux-mêmes reliés à des électrodes formées sur la partie extérieure de l'embase et aptes à venir en contact avec la paroi de l'organe du patient.

6. Le dispositif de la revendication 1, dans lequel la capsule (100) comporte un corps (102) en forme de surface de révolution dont l'axe est orienté parallèlement à l'axe des moyens d'ancrage (204),
et dans lequel le corps comporte, sur sa face tournée du côté de l'embase, au moins un support saillant (108, 128) portant une surface d'électrode (22, 24) apte à venir en contact avec les tissus du patient lorsque la capsule est montée sur l'embase.

7. Le dispositif de la revendication 6, dans lequel une paroi latérale du support saillant (108) est apte à venir en contact avec un rebord (210) de la platine de manière à assurer un blocage en rotation axiale de la capsule par rapport à l'embase.

8. Le dispositif de la revendication 6, dans lequel le corps (102) comporte, sur sa face opposée à celle tournée du côté de l'embase, un couvercle (104) portant une surface conductrice (112) formant électrode de masse, apte à venir en contact avec un fluide corporel du patient lorsque la capsule est montée sur l'embase.

9. Le dispositif de la revendication 6, dans lequel le corps (112) comporte, sur sa face opposée à celle tournée du côté de l'embase, des moyens de fixation à un élément additionnel (102') séparable du corps sans désolidarisation du corps et de l'embase, la capsule étant formée par l'empilement (102, 102') du corps (102) et de l'élément additionnel (102').

10. Le dispositif de la revendication 9, dans lequel l'élément additionnel (102') loge une batterie d'alimentation des circuits électroniques logés dans le corps (102).

11. Le dispositif de la revendication 1, dans lequel la capsule comporte un corps (116) en forme de surface de révolution dont l'axe (D₁) est orienté perpendiculairement à l'axe (D₂) des moyens d'ancrage, et dans lequel le corps comporte, sur au moins une région d'extrémité axiale, une bague annulaire (120) portant une surface d'électrode (22, 24) apte à venir en contact avec les tissus du patient lorsque la capsule est montée sur l'embase.

12. Le dispositif de la revendication 1, dans lequel la capsule (100) et/ou l'embase (200) sont pourvus de marqueurs radio-opaques.

## Claims

1. An autonomous intracorporeal active medical device (12, 14), including means (204) for anchoring to a patient's organ wall, comprising two distinct elements reversibly fastenable to each other and separable from each other, with:
- a sealed capsule (100) accommodating electronic circuits (110), and
- a base (200) including a plate (202) having an outer face carrying said anchoring means (204), and an inner face forming a support for the capsule and carrying means for mechanical coupling to the capsule, **characterized in that** the means for mechanical coupling of the base to the capsule comprise protruding tabs (206) arranged at two opposite ends of the plate (202) on the inner face of the latter, said tabs (206) being substantially parallel to each other and defining on their opposite faces contact surfaces adapted to conform the shape of mating contact surfaces of the capsule.

2. The device of claim 1, wherein the tabs (206) are elastically deformable, and wherein the contact surfaces of the base include opposite concavities (208) adapted to conform the shape of convex mating contact surfaces (102) of the capsule, so as to allow a reversible snapping of the capsule to the base.

3. The device of claim 1, wherein the tabs (206) are rigid tabs, and wherein the contact surfaces of the base include an internal screw thread (224) adapted to engage with threaded mating contact surfaces (122) of the capsule, so as to allow a reversible screwing of the capsule to the base.

4. The device of claim 1, wherein the tabs are rigid tabs, and wherein the tabs include a curved notch (226, 228) adapted to engage with counterpart coupling fingers (126) of the capsule, so as to allow a reversible nesting of the bayonet fixing type of the capsule to the base,
the base further comprising means (230) for elastically biasing the capsule in the axial direction, so as to allow a locking in position of the capsule fixed to the base.

5. The device of claim 1, wherein the capsule (100) includes a body (102) provided with electrodes coupled to contacts arranged on the lower part of the base (200), said contacts being themselves connected to electrodes formed on the outer part of the base and adapted to come into contact with the patient's organ wall.

6. The device of claim 1, wherein the capsule (100) includes a body (102) shaped as a surface of revolution whose axis is oriented parallel to the axis of the anchoring means (204),
and wherein the body comprises, on its base-side face, at least one protruding support (108, 128) carrying an electrode surface (22, 24) adapted to come into contact with the patient's tissues when the capsule is mounted on the base.

7. The device of claim 6, wherein a side wall of the protruding support (108) is adapted to come into contact with an edge (210) of the plate, so as to ensure a locking in axial rotation of the capsule with respect to the base.

8. The device of claim 6, wherein the body (102) includes, on its face opposite to its base-side face, a lid (104) carrying a conductive surface (112) forming a ground electrode, adapted to come into contact with a body fluid of the patient when the capsule is mounted on the base.

9. The device of claim 6, wherein the body (102) includes, on its face opposite to its base-side face, means for fixation to an additional element (102') that can be separated from the body with separation of the body from the base, the capsule being formed by the stacking (102, 102') of the body (102) and the additional element (102').

10. The device of claim 9, wherein the additional element (102') accommodates a power supply battery for the electronic circuits accommodated in the body (102).

11. The device of claim 1, wherein the capsule comprises a body (116) shaped as a surface of revolution whose axis (D₁) is oriented perpendicular to the axis (D₂) of the anchoring means, and wherein the body comprises, over at least an axial end region, an annular ring (120) carrying an electrode surface (22, 24) adapted to come into contact with the patient's tissues when the capsule is mounted on the base.

12. The device of claim 1, wherein the capsule (100) and/or the base (200) are provided with radio-opaque markers.

## Patentansprüche

1. Autonome intrakorporale aktive medizinische Vorrichtung (12, 14), die Einrichtungen (204) zur Verankerung an einer Wand eines Organs eines Patienten aufweist, die zwei unterschiedliche, miteinander fest verbindbare und umkehrbar trennbare Elemente enthält, mit:
- einer dichten Kapsel (100), in der elektronische Schaltkreise (110) untergebracht sind, und
- einem Unterteil (200), das eine Platte (202) aufweist, die eine Außenseite, die die Verankerungseinrichtungen (204) trägt, und eine Innenseite besitzt, die einen Träger für die Kapsel bildet und Einrichtungen zur mechanischen Kopplung mit der Kapsel trägt,
**dadurch gekennzeichnet, dass** die Einrichtungen zur mechanischen Kopplung des Unterteils mit der Kapsel vorstehende Laschen (206) enthalten, die an zwei gegenüberliegenden Enden der Platte (202) auf deren Innenseite angeordnet sind, wobei diese Laschen (206) im Wesentlichen parallel sind und auf ihren einander gegenüberliegenden Seiten Kontaktflächen definieren, die sich der Form von Gegenkontaktflächen der Kapsel anpassen können.

2. Vorrichtung nach Anspruch 1, wobei die Laschen (206) elastisch verformbar sind, und wobei die Kontaktflächen des Unterteils einander gegenüberliegende Konkavitäten (208) aufweisen, die sich der Form von konvexen Gegenkontaktflächen (102) der Kapsel anpassen können, um ein umkehrbares Einrasten der Kapsel auf dem Unterteil zu erlauben.

3. Vorrichtung nach Anspruch 1, wobei die Laschen (206) steife Laschen sind, und wobei die Kontaktflächen des Unterteils ein Innengewinde (224) aufweisen, das mit Gegengewindekontaktflächen (122) der Kapsel in Eingriff kommen kann, um ein umkehrbares Verschrauben der Kapsel auf dem Unterteil zu erlauben.

4. Vorrichtung nach Anspruch 1, wobei die Laschen steife Laschen sind, und wobei die Laschen eine gekrümmte Aussparung (226, 228) aufweisen, die mit entsprechenden Kopplungsfingern (126) der Kapsel in Eingriff kommen kann, um ein umkehrbares Einstecken von der Art Bajonett-Befestigung der Kapsel auf dem Unterteil zu erlauben,
wobei das Unterteil außerdem Einrichtungen (230) zur elastischen Beanspruchung der Kapsel in axialer Richtung aufweist, um eine Positionsverriegelung der auf dem Unterteil befestigten Kapsel zu erlauben.

5. Vorrichtung nach Anspruch 1, wobei die Kapsel (100) einen Körper (102) aufweist, der mit Elektroden versehen ist, die mit Kontakten gekoppelt sind, welche auf dem inneren Bereich des Unterteils (200) angeordnet sind, wobei diese Kontakte selbst mit Elektroden verbunden sind, die auf dem äußeren Bereich des Unterteils geformt sind und mit der Wand des Organs des Patienten in Kontakt kommen können.

6. Vorrichtung nach Anspruch 1, wobei die Kapsel (100) einen Körper (102) in Form einer drehsymmetrischen Fläche aufweist, dessen Achse parallel zur Achse der Verankerungseinrichtungen (204) ausgerichtet ist,
und wobei der Körper auf seiner zum Unterteil weisenden Seite mindestens einen vorstehenden Träger (108, 128) aufweist, der eine Elektrodenfläche (22, 24) trägt, die mit den Geweben des Patienten in Kontakt kommen kann, wenn die Kapsel auf das Unterteil montiert ist.

7. Vorrichtung nach Anspruch 6, wobei eine Seitenwand des vorstehenden Trägers (108) mit einer Randleiste (210) der Platte in Kontakt kommen kann, um eine Blockierung der axialen Drehung der Kapsel bezüglich des Unterteils zu gewährleisten.

8. Vorrichtung nach Anspruch 6, wobei der Körper (102) auf seiner der zum Unterteil weisenden Seite entgegengesetzten Seite einen Deckel (104) aufweist, der eine leitende Fläche (112) trägt, die eine Massenelektrode formt, welche mit einem Körperfluid des Patienten in Kontakt kommen kann, wenn die Kapsel auf das Unterteil montiert ist.

9. Vorrichtung nach Anspruch 6, wobei der Körper (102) auf seiner der zum Unterteil weisenden Seite entgegengesetzten Seite Einrichtungen zur Befestigung an einem zusätzlichen Element (102') aufweist, das ohne Lösen des Körpers und des Unterteils voneinander vom Körper getrennt werden kann, wobei die Kapsel durch die Stapelung (102, 102') des Körpers (102) und des zusätzlichen Elements (102') gebildet wird.

10. Vorrichtung nach Anspruch 9, wobei das zusätzliche Element (102') eine Versorgungsbatterie der im Körper (102) untergebrachten elektronischen Schaltkreise aufnimmt.

11. Vorrichtung nach Anspruch 1, wobei die Kapsel einen Körper (116) in Form einer drehsymmetrischen Fläche aufweist, dessen Achse (D₁) lotrecht zur Achse (D₂) der Verankerungseinrichtungen ausgerichtet ist, und wobei der Körper in mindestens einer axialen Endregion einen Ring (120) aufweist, der eine Elektrodenfläche (22, 24) trägt, die mit den Geweben des Patienten in Kontakt kommen kann, wenn die Kapsel auf das Unterteil montiert ist.

12. Vorrichtung nach Anspruch 1, wobei die Kapsel (100) und/oder das Unterteil (200) mit röntgendichten Markern versehen sind.
